# EUROPEAN PATENT APPLICATION

(11) **EP 1 760 769 A1**
(43) Date of publication of application: **07.03.2007**
(21) Application number: 05743293.2
(22) Date of filing: 30.05.2005
(51) Int. Cl.: H01L 21/3065

(54) **DRY ETCHING GASES AND METHOD OF DRY ETCHING**

(30) Priority: 31.05.2004 JP 2004161002; 31.05.2004 JP 2004161148
(71) Applicant: National Institute of Advanced Industrial Science and Technology, Tokyo 100-8921 (JP); ZEON CORPORATION, Chiyoda-ku Tokyo 100-8323 (JP)
(72) Inventor: Sekiya, A., Nat. Inst. of Adv. Ind. Science&Tech., Tsukuba-shi, Ibaraki 3058565 (JP); Sugimoto, Tatsuya, Chiyoda-ku, Tokyo 1008323 (JP); Yamada, Toshiro, Chiyoda-ku, Tokyo 1008323 (JP); Mase, Takanobu, Chiyoda-ku, Tokyo 1008323 (JP)
(74) Representative: Albrecht, Thomas
(86) International application number: PCT/JP2005/009865
(87) International publication number: WO 2005/117082

(57) **Abstract**

A dry etching gas comprising a C₄₋₆ fluorine compound which has an ether bond or carbonyl group and one or more fluorine atoms in the molecule and is constituted only of carbon, fluorine, and oxygen atoms and in which the ratio of the number of fluorine atoms to the number of carbon atoms (F/C) is 1.9 or lower (provided that the compound is neither a fluorine compound having one cyclic ether bond and one carbon-carbon double bond nor a saturated fluorine compound having one carbonyl group); a mixed dry etching gas comprising the dry etching gas and at least one gas selected from the group consisting of rare gases, O₂, O₃, CO, CO₂, CHF₃, CH₂F₂, CF₄, C₂F₆, and C₃F₈; and a method of dry etching which comprises converting either of these dry etching gases into a plasma and processing a semiconductor material with the plasma. The dry etching gases can be safely used, are reduced in influence on the global environment, and can highly selectively dry-etch a semiconductor material at a high dry etching rate to form a satisfactory pattern shape. The dry etching method employs either of these dry etching gases.

## Description

### TECHNICAL FIELD

The present invention relates to a dry etching gas useful for manufacturing a semiconductor device, and to a dry etching method using the dry etching gas.

### BACKGROUND ART

In recent years, the degree of integration and the performance of semiconductor devices has been remarkably improved, as is apparent from very-large-scale integrated circuits (VLSI), ultra-large-scale integrated circuits (ULSI), and the like. With such a recent trend, the technical requirements for a dry etching gas used in manufacturing a semiconductor device have become stricter than ever.
Saturated fluorocarbons such as tetrafluorocarbon and octafluorocyclcobutane have been used mainly as the dry etching gas. However, since these gases have an extremely long life exceeding several thousands of years in the air, the influence of these gases on global warming is a concern. Therefore, use of these gases tends to be restricted. To replace these gases, fluorine-containing compounds having a carbon-carbon double bond or a carbon-carbon triple bond in the molecule, such as hexafluoro-1, 3-butadiene, hexafluoro-2-butyne, and octafluorocyclopentene, have been developed.

However, when these fluorine-containing compounds are used to dry etch a silicon compound layer represented by a silicon oxide layer, selectivity of these compounds for a protective film serving as a mask such as a photoresist or polysilicon may be lowered depending on the dry etching conditions. In particular, when forming a pattern of a size of 100 nm or less, it was difficult to highly selectively form a pattern with a high aspect ratio at a high speed because a resist used as a mask is limited.

On the other hand, attempts have been made to apply fluorine-containing compounds containing an oxygen atom to dry etching. For example, JP-A-10-27781, JP-A-11-140441, JP-A-10-223614, and WO2002/66452 propose hexafluoropropene oxide (C₃F₆O), hexafluoromethyl vinyl ether (C₃F₆O), octafluoroethyl vinyl ether (C₄F₈O), decafluoropropyl vinyl ether (C₅F₁₀O), five-membered heterocyclic compound having a cyclic ether structure and a fluorine atom as ether bond-containing fluorine compounds for dry etching. JP-A-6-163476, JP-A-10-27781, and JP-T-2004-536448 propose using hexafluoroacetone (CF₃COCF₃), trifluoroacetyl fluoride (CF₃COF), and perfluorocyclopentanone (saturated heterocyclic compound) as dry etching gases.
However, the dry etching gases described in these documents have problems in which selectivity for a resist is not sufficient.

JP-A-2002-134479 discloses a technique of using octafluorocyclobutane (C₄F₈) or octafluorocyclopentene (C₅F₈) as a dry etching gas and adding carbon monoxide to the dry etching gas to attain a high aspect ratio. However, the above technique has a problem in which high costs are required to use the technique on an industrial basis.

### DISCLOSURE OF THE INVENTION

### Problems to be Solved by the Invention

The present invention has been made considering the problems of the background art. An object of the present invention is to provide a dry etching gas which can be safely used, has low environmental influence, and can achieve highly selective dry etching of a semiconductor material at a high dry etching rate to form a satisfactory pattern shape, and a dry etching method using the dry etching gas.

### Means for Solving the Problem

The inventors of the present invention made hard studies to achieve the above object, and have found that dry etching can be performed at a high dry etching rate and a high degree of selectivity using a fluorine-containing compound having a specific molecular structure as a dry etching gas. The present invention has been made based on this novel finding.

Specifically, the present invention provides the following items.
(1) A dry etching gas comprising a C₄₋₆ fluorine-containing compound which has an ether bond or a carbonyl group, and a fluorine atom, in the molecule, consists of carbon, fluorine, and oxygen atoms, and has a ratio of the number of fluorine atoms to the number of carbon atoms (F/C) of 1.9 or less, wherein the compound excludes a fluorine-containing compound having one cyclic ether bond and one carbon-carbon double bond, and a saturated fluorine-containing compound having one carbonyl group.
(2) The dry etching gas according to (1), wherein the C₄₋₆ fluorine-containing compound has a carbon-carbon unsaturated bond.
(3) The dry etching gas according to (1), wherein the C₄₋₆ fluorine-containing compound has a cyclic ether structure.
(4) A mixed dry etching gas comprising at least one gas selected from the group consisting of rare gases, O₂, O₃, CO, CO₂, CHF₃, CH₂F₂, CF₄, C₂F₆, and C₃F₈, and the dry etching gas according to (1).
(5) A dry etching method comprising the steps of: generating plasma using the dry etching gas according to (1) and processing a semiconductor material with the plasma.
(6) The dry etching method according to (5), wherein the semiconductor material is a silicon oxide.
(7) A dry etching method comprising the steps of: generating plasma using the mixed dry etching gas according to (4) and processing a semiconductor material with the plasma.
(8) The dry etching method according to (7), wherein the semiconductor material is a silicon oxide.
(9) A method of manufacturing a dry-etched semiconductor material comprising the step of: processing a semiconductor material using the dry etching method according to (5) or (7).
(10) A dry-etched semiconductor material obtained by the method according to (9).
(11) A semiconductor device comprising the dry-etched semiconductor material according to (10).

### Effect of the Invention

It is estimated that dry etching using the dry etching gas of the present invention promotes formation of a fluorocarbon film having a strong interatomic bond, to allow a highly plasma-resistant polymer film to be formed on a semiconductor material (substrate to be etched). Therefore, dry etching can be performed with a high degree of selectivity and a high aspect ratio. In particular, a hole with a satisfactory shape can be formed when forming a contact hole.
The dry etching gas of the present invention can be used safely. In addition, the dry etching gas of the present invention has little environmental influence due to its relatively short life in the air.

### BEST MODE FOR CARRYING OUT THE INVENTION

### 1) Dry etching gas

The dry etching gas of the present invention includes (A) a fluorine-containing compound having an ether bond (-O-) and a fluorine atom in the molecule (hereinafter referred to as "fluorine-containing compound A") or (B) a fluorine-containing compound having a carbonyl group and a fluorine atom in the molecule (hereinafter referred to as "fluorine-containing compound B").

The fluorine-containing compound A and the fluorine-containing compound B may be respectively used singly or in combination of two or more. Compounds given below may also be respectively used either singly or in combination of two or more.

### (A) Fluorine-containing compound A

The fluorine-containing compound A is a C₄₋₆ fluorine-containing compound which has an ether bond and a fluorine atom in the molecule, consists of carbon, fluorine, and oxygen atoms, and has a ratio of the number of fluorine atoms to the number of carbon atoms (F/C) of 1.9 or less, wherein the compound excludes a fluorine-containing compound having one cyclic ether bond and one carbon-carbon double bond.

Specific examples of the fluorine-containing compound A include:
(A-1) a C₄₋₆ fluorine-containing compound which has an ether bond and a fluorine atom in the molecule, consists of carbon, fluorine, and oxygen atoms, and has a ratio of the number of fluorine atoms to the number of carbon atoms (F/C) of 1.9 or less, in which the ether bond forms a linear ether structure;
(A-2) a C₄₋₆ fluorine-containing compound which has an ether bond and a fluorine atom in the molecule, consists of carbon, fluorine, and oxygen atoms, and has a ratio of the number of fluorine atoms to the number of carbon atoms (F/C) of 1.9 or less, in which the ether bond is an oxide ether bond;
(A-3) a C₄₋₆ fluorine-containing compound which has one ether bond, two or more carbon-carbon double bonds, and a fluorine atom in the molecule, consists of carbon, fluorine, and oxygen atoms, and has a ratio of the number of fluorine atoms to the number of carbon atoms (F/C) of 1.9 or less, in which the ether bond forms a cyclic ether structure;
(A-4) a C₄₋₆ fluorine-containing compound which has one ether bond, a carbon-carbon triple bond, or a fluorine atom in the molecule, consists of carbon, fluorine, and oxygen atoms, and has a ratio of the number of fluorine atoms to the number of carbon atoms (F/C) of 1.9 or less, in which the ether bond forms a cyclic ether structure; and
(A-5) a C₄₋₆ fluorine-containing compound which has two or more ether bonds and a fluorine atom in the molecule, consists of carbon, fluorine, and oxygen atoms, and has a ratio of the number of fluorine atoms to the number of carbon atoms (F/C) of 1.9 or less, in which the ether bond forms a cyclic ether structure.

The term "oxide ether bond" refers to a bond structure in which two adjacent carbon atoms are directly bonded and are also indirectly bonded through one oxygen atom, as represented by ethylene oxide.
The term "ether bond forming a cyclic ether structure" refers to an ether bond constituting a cyclic ether structure other than an oxide ether bond. It is generally considered that an ether bond forming a cyclic ether structure differs from an oxide ether bond.

It is preferable that the fluorine-containing compound A is a compound having a carbon-carbon unsaturated bond(a), a compound having a cyclic ether structure(b), or a compound having an oxide ether bond (c) to minimize the influence on global warming.

The carbon-carbon unsaturated bond in (a) may be either a double bond or a triple bond. However, for the same reason as mentioned above, a compound having two or more carbon-carbon double bonds (a-1) or a carbon-carbon triple bond (a-2) is more preferable.

When two or more carbon-carbon double bonds or carbon-carbon triple bonds are present, the number of bonds is normally about 2 or 3. When two or more cyclic ether structures in (b) or oxide ether bonds in (c) are present, the number of structures or bonds is normally about 2 or 3. The same can be applied to the case where the ether bond forms a linear ether structure. In the case of the cyclic ether structure or the linear ether structure, a structural unit containing one ether bond is counted as one cyclic ether structure or one linear ether structure.

As compared with the five-membered heterocyclic compound having a cyclic ether structure and a fluorine atom in the molecule described in the above-mentioned WO2002/66452, the fluorine-containing compound A has an advantage of being capable of dry etching a semiconductor material with a high degree of selectivity at a high dry etching rate to form a pattern having a satisfactory shape. In respect of the above-mentioned effects, the fluorine-containing compound A is preferably the fluorine-containing compounds (A-1) to (A-5), more preferably the fluorine-containing compounds (A-2) to (A-5), and still more preferably the fluorine-containing compound (A-3) or (A-4).

The ratio of the number of fluorine atoms to the number of carbon atoms (F/C) in the fluorine-containing compound A is 1.9 or less, preferably 1.8 or less, more preferably 1.7 or less, still more preferably 1.6 or less, and particularly preferably 1.5 or less.
If the ratio of the number of fluorine atoms to the number of carbon atoms (F/C) is too large, the generation ratio of CF₂ or CF radicals, which are precursors necessary for forming a protective film containing a carbon-fluorine bond on a substrate to be etched, is lowered, which may result in a decreased aspect ratio.

Specific examples of the above compound (a-1) include compounds having four carbon atoms such as bis(trifluorovinyl) ether, tetrafluorofuran, and 2,3,5,6-tetrafluoro-1,4-dioxin; compounds having five carbon atoms such as 2,3,4,4,5,6-hexafluoro-4H-pyran, 1,1,2,3,4-pentafluoro-4-trifluoromethoxy-1,3-butadiene, and 2-difluoromethylene-4-fluoro-5-(trifluoromethyl)-1,3-dioxole; and compounds having six carbon atoms such as bis(pentafluoroallyl ether), 2,5-bis(trifluoromethyl)furan, 2,3-bis(trifluoromethyl)furan, 2,3-bis(trifluoromethoxy)-1,1,4,4-tetrafluoro-1,3-butadiene, and bis(trifluorovinyl)-tetrafluoroethylene glycol.

Of these, the compounds having four carbon atoms or the compounds having five carbon atoms are preferable, since the ratio of the number of fluorine atoms to the number of carbon atoms can be reduced. Bis(trifluorovinyl) ether, tetrafluorofuran, 2, 3, 5, 6-tetrafluoro-1, 4-dioxin, 2,3,4,4,5,6-hexafluoro-4H-pyran, 1, 1, 2, 3, 4-pentafluoro-4-trifluoromethoxy-1, 3-butadiene, or 2-difluoromethylene-4-fluoro-5-(trifluoromethyl)-1, 3-dioxole is more preferable. Bis(trifluorovinyl) ether or tetrafluorofuran is still more preferable, with bis(trifluorovinyl) ether being particularly preferable.

The above compounds are known compounds, and can be appropriately synthesized with reference to common literature relating to synthetic organic chemistry (for example, Chemistry of Organic Fluorine Compounds (Milos Hudlicky, published by John Willy & Sons Inc., 1976) and Chemistry of Organic Fluorine Compounds II (Milos Hudlicky and Attila E. Pavlath, published by the American Chemical Society, 1995). It is preferred that bis(trifluorovinyl) ether be produced by the method described in Dutch Patent No. 6605656 (Chemical Abstracts, Vol. 66, 65088s).

Specific examples of the compound (a-2) include compounds having four carbon atoms such as 3, 3, 3-trifluoro-1-(trifluoromethoxy)-1-propyne; compounds having five carbon atoms such as 3, 3, 3-trifluoro-1-(pentafluoroethoxy)-1-propyne and 3,3,3-trifluoropropynyl-trifluorovinyl ether; and compounds having six carbon atoms such as 1,4-bis(trifluoromethoxy)-1,1,4,4-tetrafluoro-2-butyne and bis(3,3,3-trifluoropropynyl) ether.

Of these, the compounds having four carbon atoms or the compounds having five carbon atoms are preferable, since the ratio of the number of fluorine atoms to the number of carbon atoms can be reduced. 3,3,3-trifluoro-1-(trifluoromethoxy)-1-propyne, 3,3,3-trifluoro-1-(pentafluoroethoxy)-1-propyne, or 3,3,3-trifluoropropynyl-trifluorovinyl ether is more preferable, with 3,3,3-trifluoro-1-(trifluoromethoxy)-1-propyne or 3,3,3-trifluoro-1-(pentafluoroethoxy)-1-propyne being particularly preferable.

Similar to the compound (a-1), the compound (a-2) is also a known compound, and can be appropriately synthesized in the same manner as the compound (a-1). It is preferred that 3,3,3-trifluoro-1-(trifluoromethoxy)-1-propyne be produced by the method described in Journal of Chemical Society, 1978, Vol. 43, page 43.

Specific examples of the compound (b) include compounds having four carbon atoms such as 2, 3, 5, 6-tetrafluoro-1, 4-dioxin, tetrafluorofuran, 2,4,5-trifluoro-2-(trifluoromethyl)-1,3-dioxole, and 2, 2, 3, 3, 5, 6-hexafluoro-2, 3-dihydro-1, 4-dioxin; compounds having five carbon atoms such as 2,3,4,4,5,6-hexafluoro-4H-pyran, 2-difluoromethylene-4-fluoro-5-(trifluoromethyl)-1, 3-dioxole, 2,2,3,4,5-pentafluoro-2,5-dihydro-5-(trifluoromethyl)-furan, 2,2,3,3,4-pentafluoro-2,3-dihydro-5-(trifluoromethyl)-furan, 2-(difluoromethylene)-4,4,5-trifluoro-5-(trifluoromethyl)-1,3-dioxolane, 2,2-difluoro-4,5-bis(trifluoromethyl)-1,3-dioxole, 2-difluoromethylene-4, 5-difluoro-1, 3-dioxole, and 4,5-difluoro-2,2-bis(trifluoromethyl)-1,3-dioxole; and compounds having six carbon atoms such as 2-pentafluoroethoxy-pentafluoro-(3, 4-dihydro-2H-pyran), 2-trifluoromethoxy-heptafluoro-(3, 4-dihydro-2H-pyran), 4-trifluoromethoxy-heptafluoro-(5, 6-dihydro-2H-pyran), 2,3-bis(trifluoromethoxy)furan, and 2, 5-bis(trifluoromethoxy)furan.

Of these, the compounds having four carbon atoms or the compounds having five carbon atoms are preferable, since the ratio of the number of fluorine atoms to the number of carbon atoms can be reduced. 2,3,5,6-tetrafluoro-1,4-dioxane, tetrafluorofuran, 2,4,5-trifluoro-2-(trifluoromethyl)-1,3-dioxole, 2,2,3,3,5,6-hexafluoro-2,3-dihydro-1,4-dioxin, 2,3,4,4,5,6-hexafluoro-4H-pyran, 2-difluoromethylene-4-fluoro-S-(trifluoromethyl)-1,3-dioxole, 2,2,3,4,5-pentafluoro-2,5-dihydro-5-(trifluoromethyl)-furan, 2,2,3,3,4-pentafluoro-2,3-dihydro-5-(trifluoromethyl)-furan, 2-(difluoromethylene)-4,4,5-trifluoro-5-(trifluoromethyl)-1,3-dioxolane, 2,2-difluoro-4,5-bis(trifluoromethyl)-1,3-dioxole, or 4,5-difluoro-2,2-bis(trifluoromethyl)-1,3-dioxole is more preferable, with tetrafluorofuran being particularly preferable.

Specific examples of the compound (c) include compounds having four carbon atoms such as bis(trifluoromethyl)oxirene and hexafluoro-1, 3-butadiene monoxide; compounds having five carbon atoms such as hexafluorocyclopentadiene monoxide, hexafluoro-2-pentyne-4, 5-epoxide, pentafluoroethyl-trifluoromethyloxirene, octafluoro-2-pentene-4,5-epoxide, octafluoro-1-pentene-4,5-epoxide, and octafluoro-1-pentene-3, 4-epoxide; and compounds having six carbon atoms such as bis(pentafluoroethyl)oxirene and heptafluoropropyl-trifluoromethyloxirene.

Of these, the compounds having four carbon atoms or the compounds having five carbon atoms are preferable, since the ratio of the number of fluorine atoms to the number of carbon atoms can be reduced. Bis(trifluoromethyl)oxirene, hexafluoro-1,3-butadiene monoxide, hexafluorocyclopentadiene monoxide, hexafluoro-2-pentyne-4,5-epoxide, pentafluoroethyl-trifluoromethyl oxirene, octafluoro-2-pentene-4,5-epoxide, octafluoro-1-pentene-4,5-epoxide, or octafluoro-1-pentene-3,4-epoxide is more preferable, with hexafluoro-1,3-butadiene monoxide or octafluoro-2-pentene-4,5-epoxide being particularly preferable.

The compound (c) is also a known compound, and can be appropriately synthesized in the same manner as the compound (a-1) or the like. It is preferred that octafluoro-2-pentene-4,5-epoxide be produced by the method described in French Patent No. 1479871.

### (B) Fluorine-containing compound B

The fluorine-containing compound B is a C₄₋₆ fluorine-containing compound which has a carbonyl group and a fluorine atom in the molecule, consists of carbon, fluorine, and oxygen atoms, and has a ratio of the number of fluorine atoms to the number of carbon atoms (F/C) of 1.9 or less, wherein the compound excludes a saturated fluorine-containing compound having one carbonyl group.

More specific examples of the fluorine-containing compound B include:
(B-1) a C₄₋₆ fluorine-containing compound which has one carbonyl group, a carbon-carbon unsaturated bond, and a fluorine atom in the molecule, consists of carbon, fluorine, and oxygen atoms, and has a ratio of the number of fluorine atoms to the number of carbon atoms (F/C) of 1.9 or less; and
(B-2) a C₄₋₆ fluorine-containing compound which has two or more carbonyl groups and a fluorine atom in the molecule, consists of carbon, fluorine, and oxygen atoms, and has a ratio of the number of fluorine atoms to the number of carbon atoms (F/C) of 1.9 or less.

It is preferred that the fluorine-containing compound B is a compound having a carbon-carbon unsaturated bond (m), a compound having two or more carbonyl groups(n) to minimize the influence on global warming.

The carbon-carbon unsaturated bond in (m) may be either a double bond or a triple bond. The number of double bonds or triple bonds may be one or more. The number of carbon-carbon unsaturated bonds in the fluorine-containing compound B is not particularly restricted, but normally about 1 to 3. When two or more carbonyl groups are present in the fluorine-containing compound B, the number of the carbonyl groups is not particularly restricted, but normally about 2.

As compared with perfluorocyclopentanone described in the above-mentioned JP-T 2004-536448, the fluorine-containing compound B has an advantage of being capable of dry etching a semiconductor material with a high degree of selectivity at a high dry etching rate to form a pattern having a satisfactory shape. In respect of the above-mentioned effects, the fluorine-containing compound B is preferably the compound (B-1) or (B-2), and more preferably the compound (B-2).

The ratio of the number of fluorine atoms to the number of carbon atoms (F/C) in the fluorine-containing compound B is 1.9 or less, preferably 1.8 or less, more preferably 1.7 or less, still more preferably 1.5 or less, and particularly preferably 1.3 or less.
If the ratio of the number of fluorine atoms to the number of carbon atoms (F/C) is too large, when a dry etching is carried out, the generation ratio of CF₂ or CF radicals, which are precursors necessary for forming a protective film containing a carbon-fluorine bond on a substrate to be etched, is lowered, which may result in a decreased aspect ratio.

Specific examples of the compound having the carbon-carbon unsaturated bond (m) include compounds having four carbon atoms such as tetrafluorocyclobutenone, hexafluoromethyl vinyl ketone, bis(trifluoromethyl) ketene, tetrafluorodiketene, and hexafluoromethyl acrylate; compounds having five carbon atoms such as tetrafluorocyclopentadienone, hexafluoro-2-cyclopentenone, hexafluoro-3-cyclopentenone, hexafluorodivinyl ketone, hexafluoro-3-pentyn-2-one, octafluoro-3-penten-2-one, octafluoroisopropenyl acetate, octafluoroethyl acrylate, tetrafluorocyclopentene-1,3-dione, trifluoromethyl trifluoroacetyl ketene, and octafluoromethyl ethyl ketene; and compounds having six carbon atoms such as octafluoro-2-cyclohexenone, octafluoro-3-cyclohexenone, octafluoro-4-hexyn-3-one, octafluoro-3-hexyn-2-one, decafluoro-3-hexen-2-one, and decafluoro-4-hexen-3-one.

Of these, the compounds having four carbon atoms or the compounds having five carbon atoms are preferable, since the ratio of the number of fluorine atoms to the number of carbon atoms can be reduced. Tetrafluorocyclobutenone, hexafluoromethyl vinyl ketone, tetrafluorocyclopentadienone, hexafluoro-2-cyclopentenone, hexafluoro-3-cyclopentenone, hexafluorodivinyl ketone, or hexafluoro-3-pentyn-2-one is more preferable, with hexafluoro-3-pentyn-2-one or tetrafluorocyclobutenone being particularly preferable.

The above compounds are known compounds, and can be appropriately synthesized in the same manner as the fluorine-containing compound A. It is preferred that tetrafluorocyclobutenone be produced by the method described in Dokl. Akad. Nauk. SSSR. 1977, Vol. 235, page 103. It is preferred that hexafluoro-3-pentyn-2-one be produced by the method described in Synthesis, 1984, Vol. 11, page 924.

In the former method, commercially available hexafluorocyclobutene and an alcohol such as benzyl alcohol are subjected to addition-elimination reaction in the presence of a base to obtain a corresponding alkoxide, and the resulting alkoxide is heated to obtain tetrafluorocyclobutenone.
In the latter method, ethyl trifluoroacetate and triphenylmethyl phosphonium bromide are reacted in the presence of a base to obtain trifluoroacetylmethylene triphenylphosphonate. The trifluoroacetylmethylene triphenylphosphonate is reacted with trifluoroacetyl chloride, and the resulting product is treated at a high temperature under high vacuum, whereby the target product, hexafluoro-3-pentyn-2-one, can be obtained in a high yield.

Specific examples of the compound having carbonyl groups (n) include compounds having four carbon atoms such as tetrafluorocyclo-1, 2-butanedione and hexafluoro-2, 3-butanedione; compounds having five carbon atoms such as hexafluoro-1, 3-cyclopentanedione, hexafluorobutan-2-one-3-ketene, octafluoro-2, 3-pentanedione, and octafluoro-2, 4-pentanedione; and compounds having six carbon atoms such as octafluoro-1, 3-cyclohexanedione, octafluoro-1, 4-cyclohexanedione, decafluoro-2, 3-hexanedione, decafluoro-2, 4-hexanedione, and decafluoro-2, 5-hexanedione.

Of these, the compounds having four carbon atoms or the compounds having five carbon atoms are preferable, since the ratio of the number of fluorine atoms to the number of carbon atoms can be reduced. Tetrafluorocyclo-1, 2-butanedione, hexafluoro-2, 3-butanedione, octafluoro-2, 3-pentanedione, or octafluoro-2, 4-pentanedione is more preferable, with terafluorocyclo-1, 2-butanedione or hexafluoro-2, 3-butanedione being particularly preferable.

The above compounds are known compounds, and can be appropriately synthesized in the same manner as described above. It is preferred that tetrafluorocyclo-1,2-butanedione be produced by the method described in United States Patent No. 3052710. It is preferred that hexafluoro-2,3-butanedione be produced by the method described in Journal of Organic Chemistry, 1965, Vol. 30, page 2472.

In the former method, tetrafluoroethylene is reacted with a methoxide such as sodium methoxide to obtain trifluoromethoxyethylene. The resulting compound is subjected to dimerization at a high temperature to obtain 1,2-dimethoxyhexafluorocyclobutane. Then, the resulting compound is treated with concentrated sulfuric acid to obtain the target product, tetrafluorocyclo-1,2-butanedione.
In the latter method, 2,3-dichlorohexafluoro-2-butene is treated with chromic acid to obtain the target product, hexafluoro-2,3-butanedione.

The dry etching gas of the present invention contains the fluorine-containing compound A and/or the fluorine-containing compound B in a total amount of normally 50 vol% or more, preferably 90 vol% or more, more preferably 95 vol% or more, still more preferably 99 vol% or more, and particularly preferably 99.9 vol % or more.

The dry etching gas of the present invention may contain other dry etching gases or other diluting gases insofar as the object of the invention is not influenced.

### 2) Mixed dry etching gas

The mixed dry etching gas of the present invention comprises the dry etching gas of the present invention and at least one gas (mixing gas) selected from the group consisting of rare gases, O₂, O₃, CO, CO₂, CHF₃, CH₂F₂, CF₄, C₂F₆, and C₃F₈.
The above-mentioned mixing gases may be used either singly or in combination of two or more.

Examples of the rare gas include helium, neon, argon, xenon, and krypton. When the rare gas is used as the mixing gas, the volume ratio of the rare gas to the dry etching gas of the present invention is preferably 2 to 200, and more preferably 5 to 150. Addition of the rare gas enables control of the concentration of etching species generated in plasma as well as control of the ion energy. The rare gases may be used either singly or in combination of two or more.

When O₂ and/or O₃ is used as the mixing gas, the volume ratio Of O₂ and/or O₃ to the dry etching gas of the present invention is preferably 0.1 to 50, and more preferably 0.5 to 30. Addition of O₂ and/or O₃ suppresses etch stop during processing a semiconductor material with plasma generated using the resulting gas.

When CO and/or CO₂ is used as the mixing gas, the volume ratio of the CO and/or CO₂ to the dry etching gas of the present invention is preferably 5 to 150, and more preferably 10 to 100. Addition of CO and/or CO₂ improves selectivity for a mask such as a resist or polysilicon during processing a semiconductor material with plasma generated using the resulting gas.

When at least one gas selected from CHF₃, CH₂F₂, CF₄, C₂F₆ and C₃F₈ is used as the mixing gas, the volume ratio of the gas(es) to the dry etching gas of the present invention is preferably 0.01 to 1, and more preferably 0.1 to 0.5. Addition of at least one gas selected from CHF₃, CH₂F₂, CF₄, C₂F₆, and C₃F₈ improves the shape of the resulting pattern after dry etching, and increases the etching rate when processing a semiconductor material with plasma generated using the resulting gas. These gases may be used either singly or in combination of two or more.

### 3) Dry etching method

The dry etching method of the present invention comprises generating plasma using the dry etching gas or the mixed dry etching gas of the present invention and processing a semiconductor material with the plasma.

The dry etching gas of the present invention is normally carried in a container such as a gas cylinder and then connected to and installed in dry etching equipment (dry etching chamber). By opening the valve of the gas cylinder, the dry etching gas of the present invention is introduced into the dry etching chamber where the gas is subjected to plasma. The plasma acts on the dry etching gas to allow dry etching to proceed.

As a plasma generator used for generating plasma using the dry etching gas, a helicon wave type generator, a high-frequency induction type generator, a parallel flat plate type generator, a magnetron type generator, a microwave type generator, and the like can be given. The helicon wave type generator, the high-frequency induction type generator, or the microwave type generator is preferably employed since a high-density plasma can be generated readily.

It is preferred that the plasma density in the dry etching method of the present invention be 10¹⁰ ions/cm³ or more, and more preferably 10¹⁰ to 10¹³ ions/cm³. If the plasma density is 10¹⁰ to 10¹³ ions/cm³, a high dry etching rate and a high degree of selectivity can be ensured, whereby a minute pattern can be formed.

In dry etching, it is preferred that the dry etching gas of the present invention be introduced into the etching chamber vacuumed so that the pressure therein is 0.0013 to 1300 Pa, and more preferably 0.13 to 1.3 Pa.

In dry etching, it is preferred that a semiconductor material (substrate to be etched) reach a temperature of 0 to 300°C, more preferably 60 to 250°C, and still more preferably 80 to 200°C. The dry etching time is normally 0.5 to 100 minutes. Since the dry etching method of the present invention allows dry etching to be performed at a high speed, the processing time can be shortened to 2 to 10 minutes, whereby productivity can be improved.

As the semiconductor material to which the dry etching method of the present invention is applied, a silicon oxide, TEOS, BPSG, PSG, SOG, an HSQ (hydrogen silsesquioxane) - based film, an MSQ (methyl silsesquioxane) - based film (HSQ and MSQ are low-organic dielectric-constant materials), a PCB-based film, an SiOC-based film, and an SiOF-based film, and the like are preferable. Of these, a silicon oxide is particularly preferable. The above-mentioned HSQ-based film, MSQ-based film, PCB-based film, SiOC-based film, and SiOF-based film may be porous films.

It is preferred that the dry etching gas be mixed with other diluting gases or the like before or after being introduced into the dry etching chamber. In the present invention, it is particularly preferable to use the mixed dry etching gas of the present invention. It is possible to use a mixed dry etching gas prepared by adding the mixing gas to the dry etching gas in advance and kept in a container such a gas cylinder. However, in order to reduce the manufacturing cost or the like, it is preferable to use a mixed dry etching gas prepared by adding the mixing gas to the dry etching gas immediately before dry etching, specifically, before or after introducing the dry etching gas into the dry etching chamber.
The addition ratio of the mixing gas to be used and the effects brought about by the addition of the mixing gas are the same as those explained above referring to the mixed dry etching gas.

According to the dry etching method of the present invention, it is possible to obtain a semiconductor material having a satisfactory pattern shape which is dry-etched with a high degree of selectivity at a high dry etching rate.

### 4) Method of manufacturing dry-etched semiconductor material, semiconductor material, and semiconductor device

The method of manufacturing a dry-etched semiconductor material of the present invention comprises processing a semiconductor material using the dry etching method of the present invention. The manufacturing method of the present invention may be carried out by performing a dry etching step in a known method of manufacturing a semiconductor material using the dry etching method of the present invention.
The present invention also provides a dry-etched semiconductor material obtained by the manufacturing method of the present invention. The semiconductor material of the present invention has a satisfactory shape pattern such as a hole pattern. Therefore, defects such as a metal wiring failure can be minimized whereby a semiconductor device can be manufactured in a high yield. The present invention also provides a semiconductor device using the dry-etched semiconductor material of present invention. The semiconductor device may be manufactured by a known method of manufacturing a semiconductor device using the semiconductor material of the present invention.

### EXAMPLES

The present invention will be described in more detail by way of examples, which should not be construed as limiting the scope of the present invention. In the following examples, "part" indicates "part by weight" unless otherwise specified.

The purity and yield of compounds were determined by gas chromatography (GC) as described below.
Apparatus: G-5000 manufactured by Hitachi Ltd.
Column: Neutrabond-1 (length: 60 m, inner diameter: 0.25 mm, thickness: 1.5 microns)
Column temperature: The temperature was kept at 40°C for the first 10 minutes, and then raised to 240°C in 10 minutes.
Injection temperature: 200°C
Carrier gas: nitrogen (flow rate: 1 ml/min)
Detector: FID
Sample amount: 1 microliter
Internal standard: n-butane

### [Production Example 1] Preparation of bis(trifluorovinyl) ether dry etching sample

### (1-a) Synthesis of FOCCF₂CF₂OCF(CF₃)COF

This compound was synthesized according to Dutch Patent No. 6605656 (Chemical Abstracts, Vol. 66, 65088s).
An autoclave made of SUS316 was thoroughly dried, and placed under an argon atmosphere. 5 parts of cesium fluoride, 50 parts of dried diethyl glycol dimethyl ether, and 90 parts of difluoromalonyl difluoride (manufactured by SynQuest Laboratories, Inc.) were placed in the autoclave. The reactor was immersed in a dry ice/acetone bath and cooled to -78°C.
98 parts of hexafluoropropene oxide (manufactured by SynQuest Laboratories, Inc.) was slowly supplied to the autoclave through a cylinder. The reaction mixture was stirred at -78°C for three hours. The reaction mixture was separated into two layers. From the valve provided at the bottom of the autoclave, only the lower layer was collected. The collected lower layer (fluorocarbon layer) was rectified at atmospheric pressure using a distillation column with a theoretical plate number of 35 (KS-type distillation column, manufactured by Toka Seiki Co., Ltd.) to obtain 122 parts of the target product, acid fluoride (FOCCF₂CF₂OCF(CF₃)COF). The yield based on difluoromalonyl difluoride was 63%.

### (1-b) Synthesis of bis(trifluorovinyl) ether

A reactor made of SUS316 was thoroughly dried, and placed under an argon atmosphere. 150 parts of dried sodium carbonate and 100 parts of the acid fluoride (FOCCF₂CF₂OCF(CF₃)COF) obtained in (1-a) were placed in the reactor. The reactor was placed in an electric furnace. The temperature of the reactor was slowly raised to a final temperature of 220°C. The generated gas was collected by a glass trap immersed in a dry ice/acetone bath, whereby 20 parts of the target product, bis(trifluorovinyl) ether, was obtained. The yield based on the acid fluoride as the raw material was 35%.

### (1-c) Purification of bis(trifluorovinyl) ether

120 parts of the collected product (bis(trifluorovinyl) ether: purity of 89 wt%) obtained by repeating (1-a) and (1-b) in Production Example 1 was placed in a round bottom flask made of glass. The collected product was then rectified at atmospheric pressure using a distillation column with a theoretical plate number of 35 (KS-type distillation column, manufactured by Toka Seiki Co., Ltd.). The temperature of the coolant at the top of the distillation column and the temperature of the fraction trap were kept at -10 to -15°C and -78°C, respectively. By the above rectification, 43 parts of bis(trifluorovinyl) ether with a purity of 99.5 wt% was obtained.
40 parts of the resulting bis(trifluorovinyl) ether (purity: 99.5 wt%) was transferred to a stainless steel cylinder. The cylinder was connected to a vacuum line through a valve, and cooled with liquid nitrogen. Freeze-pump-thaw cycling was conducted five times, whereby a dry etching sample 1 (bis(trifluorovinyl) ether: purity of 99.99 wt%) was obtained.

### [Production Example 2] Preparation of hexafluoro-3-pentyn-2-one dry etching sample

### (2-a) Synthesis of trifluoroacetylmethylenetriphenylphosphorane

This compound was synthesized according to literature (Synthesis, 1984, Vol. 11, page 924).
A reactor made of glass provided with a three-way stopcock was thoroughly dried, and placed under an argon atmosphere. 250 parts of methyltriphenylphosphonium bromide (manufactured by Tokyo Chemical Industry Co., Ltd.) and 2500 parts of dried diethyl ether were placed in the reactor. The reactor was then cooled to -78°C. 392 parts of a cyclohexane-diethyl ether solution of 1.9M phenyl lithium (manufactured by Kanto Chemical Co., Inc.) were slowly added dropwise, and the mixture was stirred for one hour. A solution obtained by dissolving 66 parts of ethyltrifluoroacetate (manufactured by Tokyo Chemical Industry Co., Ltd.) in a dried diethyl ether was slowly added dropwise to the reactor at -78°C, and the mixture was stirred for 1.5 hours. Subsequently, the temperature of the mixture was raised to room temperature. The mixture was then stirred at room temperature for a further five hours. The reactor was immersed in an ice water bath. After the addition of 1500 parts of 4.8 wt% hydrobromic acid, the precipitate was collected by filtration. The precipitate was dissolved in 450 parts of methylene chloride, washed with a 2 wt% sodium carbonate aqueous solution, then with a saturated sodium chloride aqueous solution, and dried with sodium sulfate. The methylene chloride was distilled off using a rotary evaporator, whereby a solid product was obtained.
The solid product was recrystallized from benzene to obtain 146 parts of trifluoroacetylmethylenetriphenylphosphorane. The yield based on methyltriphenylphosphonium bromide was 70%.

### (2-b) Synthesis of bis(trifluoroacetyl)methylenetriphenylphosphorane

A reactor made of glass provided with a three-way stopcock, a bubbler, and a condenser was placed under an argon atmosphere. 146 parts of trifluoroacetylmethylenetriphenylphosphorane obtained in (2-a) and 2500 parts of dried toluene were placed in the reactor. The reaction mixture was heated to 110°C with stirring. 68 parts of trifluoroacetyl chloride (manufactured by Sigma-Aldrich Corporation) was introduced into the reactor with bubbling using the bubbler, and the mixture was stirred at 110°C for four hours. After cooling the reactor to room temperature, the mixture was stirred for a further one hour. The solvent was then distilled off from the reaction mixture using a rotary evaporator. The resulting solid product was recrystallized from a mixture of methanol and water (volume ratio of 9:1) to obtain 165 parts of bis(trifluoroacetyl)methylenetriphenylphosphorane as a solid. The yield based on trifluoroacetylmethylenetriphenylphosphorane was 89%.

### (2-c) Preparation of hexafluoro-3-pentyn-2-one

165 parts of the resulting bis(trifluoroacetyl)methylenetriphenylphosphorane was placed in a container made of SUS304. The pressure inside the container was reduced to 267 Pa using a vacuum pump. The container was placed in an electric furnace, and slowly heated to 200°C. The generated gas was collected using a glass trap immersed in a dry ice/acetone bath, whereby 48 parts of the target product, hexafluoro-3-pentyn-2-one (purity: 92 wt%), was obtained. The yield based on bis(trifluoroacetyl)methylenetriphenylphosphorane was 72%.

### (2-d) Purification of hexafluoro-3-pentyn-2-one

264 parts of the collected product (hexafluoro-3-pentyn-2-one: purity of 92 wt%) obtained by repeating (2-a) to (2-c) in Production Example 2 was placed in a round bottom flask made of glass. The collected product was then rectified at atmospheric pressure using a distillation column with a theoretical plate number of 35 (KS-type distillation column, manufactured by Toka Seiki Co., Ltd.). The temperature of the coolant at the top of the distillation column and the temperature of the fraction trap were kept at 15 to 20°C and -78°C, respectively. By the above rectification, 121 parts of hexafluoro-3-pentyn-2-one (boiling point: 65°C) with a purity of 99.6 wt% was obtained.
100 parts of the resulting hexafluoro-3-pentyn-2-one (purity: 99.6 wt%) was transferred to a stainless steel cylinder. The cylinder was connected to a vacuum line through a valve, and cooled with liquid nitrogen. Freeze-pump-thaw cycling was conducted four times, whereby a dry etching evaluation sample 2 (hexafluoro-3-pentyn-2-one: purity 99.99 wt%) was obtained.

### [Production Example 3] Preparation of tetrafluorocyclobutenone dry etching sample

### (3-a) Synthesis of benzyloxypentafluoro-1-cyclobutene

80 parts of benzyl alcohol and 100 parts of hexafluorocyclobutene (manufactured by SynQuest Laboratories, Inc.) were placed in a reactor made of glass. The reactor was immersed in an ice water bath. While stirring the contents inside the reactor, 41 parts of powdery potassium hydroxide was slowly added. The mixture was stirred for one hour in the ice water bath. The reactor was then allowed to warm to room temperature, and the mixture was stirred at room temperature for a further three hours. The contents were poured into 300 parts of ice water to collect an organic layer. The collected organic layer was washed with a saturated sodium chloride aqueous solution, dried with sodium sulfate, and rectified by distillation under reduced pressure, whereby 93 parts of benzyloxypenetafluoro-1-cyclobutene was obtained. The yield based on hexafluorocyclobutene was 62 %.

### (3-b) Synthesis of tetrafluorocyclobutenone

90 parts of benzyloxypentafluoro-l-cyclobutene obtained in (3-a) was placed in a container made of SUS316. The container was placed in an electric furnace, and slowly heated to 200°C. The generated gas was collected by a glass trap immersed in a dry ice/acetone bath through a tube filled with sodium fluoride pellets, whereby 36 parts of the target product, tetrafluorocyclobutenone (purity: 93%), was obtained. The yield based on benzyloxypentafluoro-1-cyclobutene was 71%.

### (3-c) Purification of tetrafluorocyclobutenone

143 parts of the collected product (tetrafluorocyclobutenone: purity of 93 wt%) obtained by repeating (3-a) and (3-b) in Production Example 3 was placed in a round bottom flask made of glass. The collected product was then rectified at atmospheric pressure using a distillation column with a theoretical plate number of 35 (KS-type distillation column, manufactured by Toka Seiki Co., Ltd.). The temperature of the coolant at the top of the distillation column and the temperature of the fraction trap were kept at 15 to 20°C and 0°C, respectively. By the above rectification, 68 parts of tetrafluorocyclobutenone (boiling point: 45°C) with a purity of 99.4 wt% was obtained.
60 parts of the resulting tetrafluorocyclobutenone (purity: 99.4 wt%) was transferred to a stainless steel cylinder. The cylinder was connected to a vacuum line through a valve, and cooled with liquid nitrogen. Freeze-pump-thaw cycling was conducted five times to obtain a dry etching evaluation sample 3 (tetrafluorocyclobutenone: purity of 99.99 wt%).

### [Example 1]

10 parts of a terpolymer obtained from 5-norbornene-2-methanol, maleic anhydride, and t-butyl methacrylate (copolymerization ratio = 1:1:1 (molar ratio), molecular weight: 5,500) and 0.2 parts of triphenyltrifluoromethane sulfonate as an acid generator were dissolved in 70 parts of propylene glycol monomethyl ether acetate. The mixture was filtered using a filter with a pore diameter of 100 nm to prepare a resist solution.
The resist solution was applied by spin coating to an 8-inch silicon substrate on which a 2-micron thick silicon oxide film was formed. The silicon substrate was prebaked on a hot plate at 120°C to form a resist film with a thickness of 450 nm. The resist film was exposed using an ArF excimer laser exposure device (exposure wavelength: 193 nm) through a mask having a contact hole pattern. Subsequently, the substrate was postbaked at 130°C, developed at 25°C for 60 seconds using a 2.38 wt% tetramethylammonium hydroxide aqueous solution, and then dried to form contact holes with a diameter of 100 nm.
The substrate on which the contact hole pattern was formed was placed in a chamber of a microwave-type plasma etching device. The inside of the reaction system was made vacuum using a pump. As the dry etching gas, the dry etching evaluation sample 1 (bis(trifluorovinyl) ether: F/C = 1.5) obtained in Production Example 1 was introduced at a rate of 50 sccm. Further, argon was supplied as an inert gas at a rate of 900 sccm and an oxygen gas was supplied at a rate of 60 sccm to keep the pressure inside the reaction system at 1.0 Pa. Dry etching was performed at a microwave power of 1200 W (2.45 GHz), a substrate bias power of 1000 W (800 KHz), and a plasma density of 10¹¹ ions/cm³.

The dry etching rate of the silicon oxide film was 576 nm/min at the center portion and 484 nm/min at the edge portion. The dry etching rate of the resist was 48 nm/min at the center portion and 61 nm/min at the edge portion. The selective ratio of dry etching was 12 at the center portion and 7.9 at the edge portion. The shape of the contact hole formed was satisfactory. The results are shown in Table 1.

### [Example 2]

Dry etching was performed in the same manner as in Example 1 except that the dry etching evaluation sample 2 (hexafluoro-3-pentyn-2-one: F/C = 1.2) obtained in Production Example 2 was used as the dry etching gas. The dry etching rate and selective ratio were determined, and the shape of the contact hole was observed. The results are shown in Table 1.

### [Example 3]

Dry etching was performed in the same manner as in Example 1 except that the dry etching evaluation sample 3 (tetrafluorocyclobutenone: F/C = 1.0) obtained in Production Example 3 was used as the dry etching gas. The dry etching rate and selective ratio were determined, and the shape of the contact hole was observed. The results are shown in Table 1.

### [Comparative Example 1]

Dry etching was performed in the same manner as in Example 1 except that commercially available hexafluoro-1, 3-butadiene (manufactured by Kanto Denka Kogyo Co., Ltd., purity: 99.9 wt%, F/C = 1.5) was used as the dry etching gas. The dry etching rate and selective ratio were determined, and the shape of the contact hole was observed. The results are shown in Table 1.

### [Comparative Example 2]

Dry etching was performed in the same manner as in Example 1 except that commercially available hexafluoropropene oxide (manufactured by SynQuest Laboratories, Inc, purity: 97 wt%, F/C = 2.0) was used as the dry etching gas. The dry etching rate and selective ratio were determined, and the shape of the contact hole was observed. The results are shown in Table 1.

### [Comparative Example 3]

Dry etching was performed in the same manner as in Example 1 except that commercially available hexafluoroacetone (manufactured by SynQuest Laboratories, Inc, purity: 97 wt%, F/C = 2.0) was used as the dry etching gas after purifying the product to a purity of 99.9 wt%. The dry etching rate and selective ratio were determined, and the shape of the contact hole was observed. The results are shown in Table 1.

The dry etching gases used were as follows.
S I : bis(trifluorovinyl) ether
S2: hexafluoro-3-pentyn-2-one
S3: tetrafluorocyclobutenone
R1: hexafluoro-1,3-butadiene
R2: hexafluoropropene oxide
R3: hexafluoroacetone

The etching rate is expressed as nm/min and the selective ratio is defined as "etching rate for silicon oxide film/etching rate for resist".

**[Table 1]**

| | | | Example 1 | Example 2 | Example 3 | Comparative Example 1 | Comparative Example 2 | Comparative Example 3 |
|---|---|---|---|---|---|---|---|---|
| Dry Etching Gas | | | S1 | S2 | S3 | R1 | R2 | R3 |
| Etching Rate | Silicon Oxide Film | Center Portion | 576 | 594 | 552 | 510 | 376 | 392 |
| | | Edge Portion | 484 | 530 | 481 | 405 | 298 | 308 |
| | Resist | Center Portion | 48 | 40 | 43 | 44 | 67 | 64 |
| | | Edge Portion | 61 | 55 | 58 | 62 | 75 | 70 |
| Selective Ratio | | Center Portion | 12.0 | 14.9 | 12.8 | 11.6 | 5.6 | 6.1 |
| | | Edge Portion | 7.9 | 9.6 | 8.3 | 6.5 | 4.0 | 4.4 |
| Shape of Contact Hole | | Center Portion | Good | Good | Good | Poor | Poor | Poor |
| | | Edge Portion | Good | Good | Good | Poor | Poor | Poor |

From Table 1, it is understood that, as compared with Comparative Example 1 using hexafluoro-1, 3-butadiene having neither an ether bond nor a carbonyl group, with Comparative Example 2 using hexafluoropropene oxide with an F/C of 2.0, and with Comparative Example 3 using hexafluoroacetone with an F/C of 2.0, the dry etching rate and selectivity were improved and a contact hole with a satisfactory shape was obtained using the dry etching gases of the examples of the present invention.

## Claims

1. A dry etching gas comprising a C₄₋₆ fluorine-containing compound which
has an ether bond or a carbonyl group, and a fluorine atom, in the molecule, consists of carbon, fluorine, and oxygen atoms, and
has a ratio of the number of fluorine atoms to the number of carbon atoms (F/C) of 1.9 or less,
wherein the compound excludes a fluorine-containing compound having one cyclic ether bond and one carbon-carbon double bond, and a saturated fluorine-containing compound having one carbonyl group.

2. The dry etching gas according to claim 1, wherein the C₄₋₆ fluorine-containing compound has a carbon-carbon unsaturated bond.

3. The dry etching gas according to claim 1, wherein the C₄₋₆ fluorine-containing compound has a cyclic ether structure.

4. A mixed dry etching gas comprising the dry etching gas according to claim 1 and at least one gas selected from the group consisting of rare gases, O₂, O₃, CO, CO₂, CHF₃, CH₂F₂, CF₄, C₂F₆, and C₃F₈.

5. A dry etching method comprising the steps of: generating plasma using the dry etching gas according to claim 1 and
processing a semiconductor material with the plasma.

6. The dry etching method according to claim 5, wherein the semiconductor material is a silicon oxide.

7. A dry etching method comprising the steps of: generating plasma using the mixed dry etching gas according to claim 4 and
processing a semiconductor material with the plasma.

8. The dry etching method according to claim 7, wherein the semiconductor material is a silicon oxide.

9. A method of manufacturing a dry-etched semiconductor material comprising the step of: processing a semiconductor material by the dry etching method according to claim 5 or 7.

10. A dry-etched semiconductor material obtained by the method according to claim 9.

11. A semiconductor device comprising the dry-etched semiconductor material according to claim 10.
